# FASCICULE DE BREVET EUROPEEN

(11) **EP 1 909 664 B1**
(45) Date de publication et mention de la délivrance du brevet: **13.06.2012**
(21) Numéro de dépôt: 06778891.9
(22) Date de dépôt: 19.07.2006
(51) Int. Cl.: A61B 17/70

(54) **DISPOSITIF INTER EPINEUX ROTATOIRE**
ROTIERENDES INTERSPINÖSES GERÄT
ROTATING INTERSPINOUS DEVICE

(30) Priorité: 21.07.2005 FR 0507747
(43) Date de publication de la demande: 16.04.2008
(73) Titulaire: Khalife, Charles, 80090 Amiens (FR)
(72) Inventeur: Khalife, Charles, 80090 Amiens (FR)
(74) Mandataire: Gasquet, Denis
(86) Numéro de dépôt international: PCT/FR2006/001773
(87) Numéro de publication internationale: WO 2007/010140

(56) Documents cités:
- WO-A-2006/106246
- DE-A1- 2 821 678
- US-A1- 2004 106 995
- DATABASE WPI Section PQ, Week 198345 Derwent Publications Ltd., London, GB; Class P31, AN 1983-813313 XP002378871 & SU 988 281 A (PUSTOVOITENKO V T) 25 janvier 1983 (1983-01-25)

## Description

La présente invention concerne un implant destiné à être posé au niveau de la colonne vertébrale, et plus particulièrement entre les apophyses épineuses afin de restituer la hauteur de l'espace discal et décomprimer les éléments nerveux. L'implant est généralement utilisé seul dans les cas d'affaissement symptomatique, ou en extrémité de montage en association avec une ostéosynthèse rachidienne, pour diminuer les contraintes supportées par l'étage adjacent à l'étage fixé.

L'invention concerne plus particulièrement des perfectionnements à ce type d'implants. Quelques modèles d'implants inter épineux existent actuellement, et malgré les améliorations développées par les fabricants, aucun de ces implants ne donne une entière satisfaction.

Généralement les implants inter épineux sont en monobloc et ne respectent pas les mouvements physiologiques de rotation tridimensionnelle de la colonne vertébrale, ce qui peut provoquer un conflit entre les apophyses épineuses et les implants. D'autres modèles possèdent plusieurs pièces en assemblage d'où risque de démontage ou de débris d'usure.

Un implant selon la préambule de la revendication 1 est connu de la demande de brevet américain US-A-2004/06995.

On connaît aussi par la divulgation faite par le brevet WO-A-20061060246, un implant chirurgical destiné à maintenir et à assister le mouvement de deux vertèbres successives entre elles, comprenant une pièce supérieur et une pièce inférieure destinées à être associées chacune à l'apophyse épineuse d'une desdites vertèbres, les pièces comprennent des moyens d'association en rotation formant ainsi une rotule. Ces moyens sont réalisés par trois éléments, un élément supérieur, un élément inférieur et un élément interposé entre les dits éléments inférieur et supérieur. Ledit élément interposé et réalisé en matière viscoélastique, la rotule fonctionne par donc par déformation de la matière.

L'implant selon l'invention permet de remédier efficacement aux deux problèmes de la rotation et du risque de démontage ou de débris d'usure.

L'implant selon l'invention est composé de deux pièces distinctes destinées à être mobiles entre elles et notamment articulées pour former une seule pièce mobile. Une vis d'assemblage peut être ajoutée au dispositif pour empêcher le déboîtement des deux pièces. Chacune des deux pièces est fixée à une apophyse épineuse d'un côté, grâce à une échancrure adaptée, et encastrée dans l'autre pièce de l'autre côté, pour former une seule pièce, articulée, possédant une mobilité tridimensionnelle.

Selon une caractéristique de l'invention, les parties des pièces d'ancrages destinées à être encastrées, l'une femelle, l'autre mâle, peuvent être de forme cylindrique pour permettre des mouvements de rotation, ou de forme sphérique pour assurer des mouvements multidirectionnels.

Selon une disposition avantageuse de l'invention les pièces d'encastrement, mâle et femelle, peuvent posséder différentes hauteurs représentant plusieurs tailles du dispositif pour s'adapter à l'espace inter épineux.

Selon une autre caractéristique avantageuse de l'invention, une vis d'assemblage peut être ajoutée pour lier les deux pièces entre elles et empêcher leur déboîtement tout en maintenant la possibilité de mouvement tridimensionnel.

Les deux pièces du dispositif peuvent être fabriquées avec différents types de matériaux biocompatibles supportant la friction, comme par exemple le titane, l'acier inoxydable, ou toute autre matière appropriée.

D'autres caracteristiques et avantages de l'invention se dégageront de la description qui va suivre en regard des dessins annexés qui ne sont donnés qu'à titre d'exemples non limitatifs.

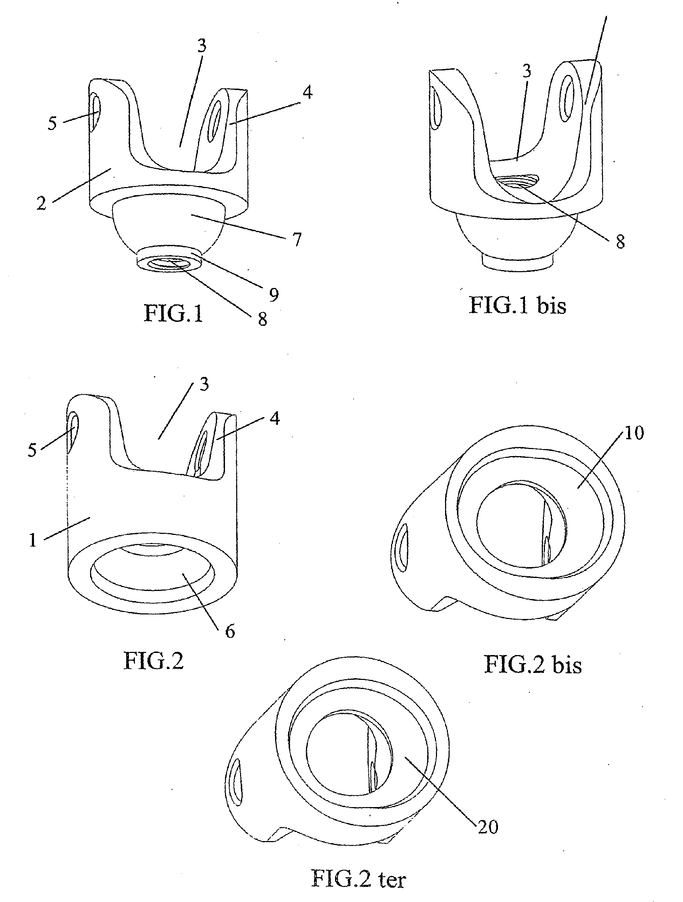

Les figures 1 à 9 représentent les différentes pièces du système selon l'invention.

Les figures 1, 1bis, 2, 2bis, 2ter, 5, et 6, représentent les différentes pièces d'un premier mode de réalisation du système selon l'invention, selon lequel la coopération entre les deux pièces d'ancrage est sphérique afin de donner une mobilité multidirectionnelle, entre les deux pièces d'ancrage.
La figure 1 est une vue de dessous en perspective de bas de la pièce d'ancrage mâle sphérique.
La figure 1 bis est une vue de dessus en perspective de la pièce d'ancrage mâle sphérique.
La figure 2 est une vue de dessous en perspective de la pièce d'ancrage femelle sphérique.
La figure 2 bis est une vue de dessous en perspective de la pièce d'ancrage femelle sphérique avec fente oblongue droite
La figure 2 ter est une vue de dessous en perspective de la pièce d'ancrage femelle sphérique avec fente oblongue en arc de cercle

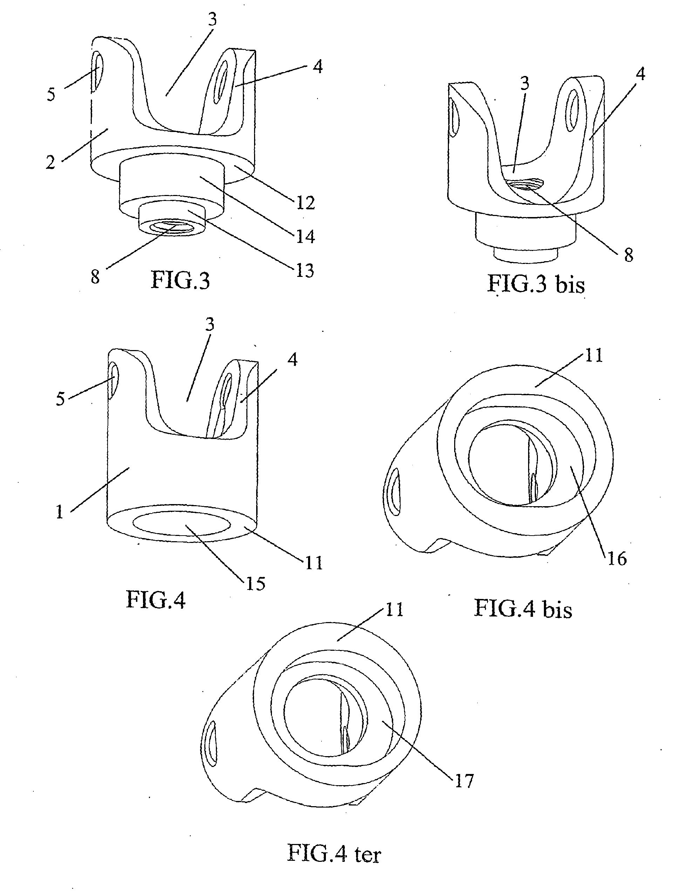

Les figures 3, 3bis, 4, 4bis, 4ter, 7, 8, et 9, représentent les différentes pièces d'un deuxième mode de réalisation du système selon l'invention, selon lequel la coopération entre les deux pièces d'ancrage est cylindrique afin de donner une mobilité en rotation, entre les deux pièces d'ancrage.
La figure 3 est une vue de dessous en perspective de la pièce d'ancrage mâle cylindrique.
La figure 3 bis est une vue de dessus en perspective de la pièce d'ancrage mâle cylindrique.
La figure 4 est une vue de dessous en perspective de la pièce d'ancrage femelle cylindrique.
La figure 4 bis est une vue de dessous en perspective de la pièce d'ancrage femelle cylindrique, avec fente oblongue droite
La figure 4 ter est une vue de dessous en perspective de la pièce d'ancrage femelle cylindrique, avec fente oblongue en arc de cercle

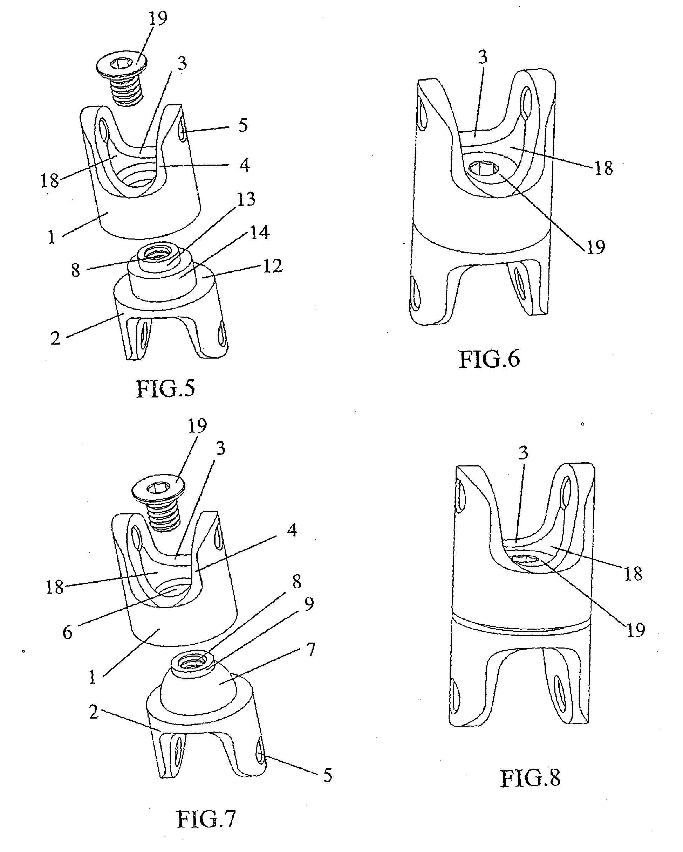

La figure 5 est une vue en perspective éclatée des deux pièces d'ancrage avec la vis d'assemblage
La figure 6 est une vue en perspective de l'assemblage des deux pièces d'ancrage cylindriques
La figure 7 est une vue des deux pièces sphériques d'ancrage avec vis d'assemblage
La figure 8 est une vue de l'assemblage des deux pièces d'ancrage sphériques

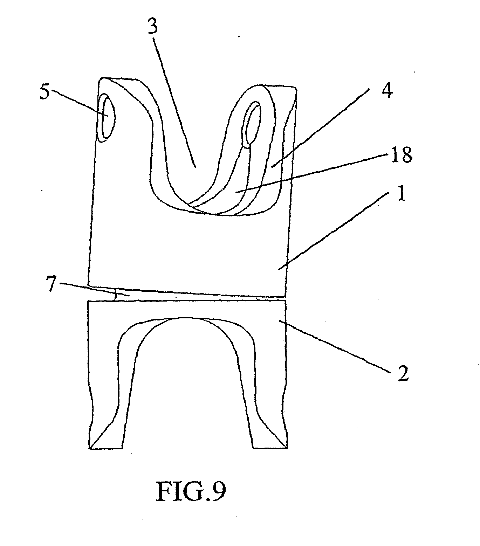

La figure 9 est une vue de l'assemblage des deux pièces d'ancrage sphériques avec inclinaison

Les dessins annexés illustrent l'invention.

On décrira ci-après, en faisant référence aux figures 1 à 9, le mécanisme de fonctionnement du dispositif inter épineux rotatoire.

Pour une description plus claire de l'invention, Les deux pièces d'ancrage du dispositif, destinées à être encastrées, seront appelées pièces d'ancrage femelle et mâle. Ces pièces d'ancrage, femelles et mâles, peuvent avoir des formes sphériques ou cylindriques.

L'implant de l'invention est caractérisée en ce qu'il comprend deux pièces d'ancrage, l'une femelle (1), l'autre mâle (2) destinées à s'ancrer aux apophyses épineuses d'un côté, et à coopérer entre elles en s'encastrant entre elles, de l'autre côté par coopération de formes, coopération de formes sphériques ou cylindriques, pour former une pièce unique, articulée, pouvant effectuer des mouvements de rotation pour l'un des mode de réalisation, voir multidirectionnels pour l'autre mode de réalisation.

Selon une caractéristique de l'invention, les deux pièces d'ancrage, sphériques ou cylindriques, femelle(1), et mâle(2), possèdent des échancrures (3) leur permettant de se fixer aux apophyses épineuses.

Selon une disposition avantageuse les échancrures (3) sont limitées par des parois verticales (4) de hauteur optimale pour pouvoir placer un deuxième dispositif au niveau de l'étage adjacent.

Selon une autre disposition avantageuse, les parois verticales (4) sont munies d'orifices (5) pour permettre d'introduire un système d'ancrage qui fixera les pièces d'ancrage femelle (1) et mâle (2) aux apophyses épineuses. Le système d'ancrage peut être de type rigide, vis ou tige, ou souple comme un fil de suture ou un ligament synthétique.

Selon une caractéristique de l'invention, la pièce d'ancrage femelle (1) est munie, du côté opposé à l'échancrure (3), d'un orifice, ou logement de coopération (6, 13), qui est sphérique (6) pour l'un des mode de réalisation ou cylindrique (15) pour l'autre des modes de réalisation, destiné à recevoir respectivement la sphère (7) ou le cylindre (14) de la pièce d'ancrage mâle (2) correspondante.

Selon une autre caractéristique de l'invention, la pièce d'ancrage mâle (2) possède, du côté opposé à l'échancrure (3), une partie sphérique (7) ou cylindrique (14) destinée à s'encastrer dans l'orifice sphérique (6) ou cylindrique (15) de la partie femelle (2), pour relier les deux pièces (1), (2), du dispositif et assurer la mobilité entre elles, et notamment la rotation et :ou le mouvement multidirectionnel.

Selon une disposition avantageuse de l'invention la partie sphérique (7) ou cylindrique (14) mâle est munie d'un taraudage (8), du côté opposé à l'échancrure (3), et pouvant déboucher dans celle-ci, pour recevoir la vis d'assemblage (19) qui va empêcher le déboîtement des deux pièces assemblées. La vis d'assemblage (19) est introduite à travers un orifice (18) réalisé au fond de l'échancrure(3)de la pièce d'ancrage femelle (1).

Selon une autre disposition avantageuse de l'invention, la distance entre les échancrures (3) des deux pièces d'ancrage (1), (2), montées l'une dans l'autre, constitue la hauteur définissant la taille du dispositif (fig6, fig8)

Selon encore une caractéristique de l'invention, la pièce d'ancrage cylindrique mâle (2) possède à la base de la partie cylindrique (14) une collerette d'appui(12) qui sert de butée à la paroi d'appui (11) de l'orifice cylindrique (15), pour définir la hauteur souhaitée du dispositif, mesurée entre les deux échancrures (3). Pour les pièces d'ancrage sphériques, la hauteur est assurée par la butée entre les parties sphériques, femelle (6) et mâle (7).

Selon une disposition avantageuse de l'invention, la distance entre l'échancrure (3) de la pièce d'ancrage cylindrique mâle (2) et la collerette (12), sur laquelle repose la paroi (11) de l'orifice cylindrique (15), peut avoir plusieurs hauteurs pour augmenter la hauteur du dispositif assemblé (fig 6) et s'adapter à l'espace inter épineux anatomique à combler. Pour la pièce d'ancrage sphérique mâle (2), la distance entre l'échancrure (3) et le dôme sphérique (7) peut avoir plusieurs hauteurs pour définir plusieurs tailles du dispositif.

Selon une autre disposition avantageuse de l'invention, la hauteur du dispositif assemblé (figures 6 et 8) peut aussi être augmentée par l'augmentation de la hauteur de la pièce d'ancrage sphérique ou cylindrique femelle (1) en dessous de l'échancrure (3).

Selon une disposition avantageuse de l'invention, les parties sphérique (7) et cylindrique (14) des pièces d'ancrage mâles (2) peuvent se terminer par un petit segment cylindrique (9, 13) destiné à servir de butée limitant l'amplitude des mouvements multidirectionnels.

## Revendications

1. Dispositif pour la chirurgie du rachis destiné à être posé en distraction dans l'espace inter épineux du type implant, qui comporte deux pièces d'encrage **caractérisé en ce qu'**il comporte deux pièces distinctes à savoir deux pièces d'ancrage, fixées chacune à une apophyse épineuse par le biais d'une échancrure (3), les dites pièces d'ancrage (1, 2) étant reliées pour être articulées entre elles, **caractérisé en ce que** lesdites pièces d'encrage étant l'une femelle (1), l'autre mâle (2), coopérant entre elles en s'encastrant entre elles par coopération de formes, grâce à deux parties cylindriques ou sphériques, l'une femelle (6, 15) et l'autre mâle (7, 14), pour assurer un mouvement rotatoire ou multidirectionnel

2. Dispositif inter épineux selon la revendication 1, **caractérisé en ce que** les pièces d'ancrage, femelle (1) et mâle (2), possèdent des échancrures adaptées (3) pour les fixer à l'apophyse épineuse

3. Dispositif inter épineux selon l'une quelconque des revendications 1 et 2, **caractérisé en ce que** la pièce d'ancrage cylindrique femelle (1) possède, du côté opposé à l'échancrure (3), un orifice cylindrique (15) destiné à recevoir la partie cylindrique mâle (14).

4. Dispositif selon l'une quelconque des revendications précédentes, **caractérisé en ce que** la pièce d'ancrage cylindrique mâle (2) possède du côté opposé à l'échancrure (3) une partie cylindrique ((4) destinée à s'encastrer dans l'orifice cylindrique (15) pour former un système rotatoire, avec possibilité de translation, entre les deux pièces cylindriques, du dispositif.

5. Dispositif selon l'une quelconque des revendications précédentes, **caractérisé en ce que** la pièce d'ancrage sphérique femelle (1) possède, du côté opposé à l'échancrure (3), un orifice sphérique (6) destiné à recevoir la partie sphérique mâle (7).

6. Dispositif selon la revendication 5, **caractérisé en ce que** la pièce d'ancrage sphèrique mâle (2) possède du côté opposé à l'échancrure (3) une partie sphérique (7) destinée à s'encastrer dans l'orifice sphérique (6) pour former un système rotatoire multidirectionnel, avec possibilité de translation, entre les deux pièces sphériques, du dispositif

7. Dispositif selon l'une quelconque des revendications précédentes, **caractérisé en ce que** les pièces d'ancrage femelles (1) ou mâles (2), cylindriques ou sphériques, possèdent plusieurs hauteurs pour former un dispositif de tailles différentes à s'adapter aux besoins anatomiques

8. Dispositif selon l'une quelconque des revendications précédentes, **caractérisé en ce que** les pièces d'ancrage femelles (1) et mâles (2), sont liées, après encastrement, par une vis d'assemblage (19) qui condamne un éventuel déboîtement, tout en maintenant les mouvements relatifs entre les deux pièces d'ancrage (1, 2).

## Claims

1. Device of the implant type for surgery of the rachis, which is designed to be fitted for the purpose of separation in the interspinous space, and comprises two anchorage parts, **characterised in that** it comprises two distinct parts, i.e. two anchorage parts which are each secured to a spiny apophysis by means of an indentation (3), the said anchorage parts (1, 2) being connected in order to be articulated to one another, **characterised in that** one of the said anchorage parts is female (1) and the other is male (2), and they co-operate with one another by being fitted together by co-operation of forms by means of two cylindrical or spherical parts, one of which is female (6, 15) and the other is male (7, 14), in order to assure rotary or multi-directional movement.

2. Interspinous device according to claim 1, **characterised in that** the female (1) and male (2) anchorage parts have indentations (3) which are designed to secure them to the spiny apophysis.

3. Interspinous device according to either of claims 1 and 2, **characterised in that** the female cylindrical anchorage part (1) has, on the side opposite the indentation (3), a cylindrical aperture (15) which is designed to receive the male cylindrical part (14).

4. Device according to any one of the preceding claims, **characterised in that** the male cylindrical anchorage part (2) has, on the side opposite the indentation (3), a cylindrical part (14) which is designed to be fitted in the cylindrical aperture (15), in order to form a rotary system, with the possibility of translation, between the two cylindrical parts of the device.

5. Device according to any one of the preceding claims, **characterised in that** the female spherical anchorage part (1) has, on the side opposite the indentation (3), a spherical aperture (6) which is designed to receive the male spherical part (7).

6. Device according to claim 5, **characterised in that** the male spherical anchorage part (2) has, on the side opposite the indentation (3), a spherical part (7) which is designed to be fitted in the spherical aperture (6), in order to form a multi-directional rotary system, with the possibility of translation, between the two spherical parts of the device.

7. Device according to any one of the preceding claims, **characterised in that** the spherical or cylindrical female (1) or male (2) anchorage parts have a plurality of heights in order to form a device with different heights which is designed to adapt to anatomical needs.

8. Device according to any one of the preceding claims, **characterised in that** the female (1) and male (2) anchorage parts (2) are connected, after being fitted together, by an assembly screw (19) which stops any dislocation of them, whilst maintaining the relative movements between the two anchorage parts (1, 2).

## Patentansprüche

1. Vorrichtung für den chirurgischen Einsatz an der Wirbelsäule, dazu bestimmt, abgesondert in den interspinösen Raum eingesetzt zu werden, vom Typ eines Implantats, umfassend zwei Verankerungselemente, **dadurch gekennzeichnet, dass** sie zwei unterschiedliche Elemente aufweist, nämlich zwei Verankerungselemente, von denen jedes mittels einer Aussparung (3) an einem Dornfortsatz angebracht ist, wobei die besagten Verankerungselemente (1, 2) gelenkig miteinander verbunden sind, **dadurch gekennzeichnet, dass** es sich bei dem einen Verankerungselement um ein weibliches (1) und bei dem anderen um ein männliches (2) Teil handelt, die zusammenwirken, indem sie sich an Hand zueinander passender Formen ineinanderfügen, mittels zweier Teile, die zylindrisch oder kugelförmig sind, von denen eines das weibliche Teil (6, 15) und das andere das männliche Teil (7, 14) ist, um eine rotierende Bewegung oder eine Bewegung in mehrere Richtungen zu gewährleisten.

2. Interspinöse Vorrichtung nach Anspruch 1, **dadurch gekennzeichnet, dass** die weiblichen (1) und männlichen (2) Verankerungselemente angepasste Aussparungen (3) besitzen, um sie am Dornfortsatz zu befestigen.

3. Interspinöse Vorrichtung nach einem der Ansprüche 1 und 2, **dadurch gekennzeichnet, dass** das weibliche zylindrische Verankerungselement (1) auf der gegenüberliegenden Seite der Aussparung (3) eine zylindrische Öffnung (15) zur Aufnahme des männlichen zylindrischen Teils (14) besitzt.

4. Vorrichtung nach einem der vorstehenden Ansprüche, **dadurch gekennzeichnet, dass** das männliche zylindrische Verankerungselement (2) auf der gegenüberliegenden Seite der Aussparung (3) ein zylindrisches Teil (14) besitzt, das dazu bestimmt ist, sich in die zylindrische Öffnung (15) einzufügen, um ein rotierendes System zu bilden, mit Translationsmöglichkeit zwischen den beiden zylindrischen Teilen der Vorrichtung.

5. Vorrichtung nach einem der vorstehenden Ansprüche, **dadurch gekennzeichnet, dass** das weibliche kugelförmige Verankerungselement (1) auf der gegenüberliegenden Seite der Aussparung (3) eine kugelförmige Öffnung (6) besitzt, die dazu bestimmt ist, das männlichen kugelförmige Teil (7) aufzunehmen.

6. Vorrichtung nach Anspruch 5, **dadurch gekennzeichnet, dass** das männliche kugelförmige Verankerungselement (2) auf der gegenüberliegenden Seite der Aussparung (3) ein kugelförmiges Teil (7) besitzt, das dazu bestimmt ist, sich in die kugelförmige Öffnung (6) einzufügen, um ein in mehrere Richtungen rotierendes System zu bilden, mit Translationsmöglichkeit zwischen den beiden kugelförmigen Teilen der Vorrichtung.

7. Vorrichtung nach einem der vorstehenden Ansprüche, **dadurch gekennzeichnet, dass** die weiblichen (1) oder männlichen (2) Verankerungselemente, die zylindrisch oder kugelförmig sind, mehrere Höhen aufweisen, um eine Vorrichtung mit unterschiedlichen Größen zu bilden, die sich an die anatomischen Erfordernisse anpassen kann.

8. Vorrichtung nach einem der vorstehenden Ansprüche, **dadurch gekennzeichnet, dass** die weiblichen (1) und männlichen (2) Verankerungselemente nach dem Einsetzen durch eine Maschinenschraube (19) verbunden sind, die ein mögliches Auskugeln verhindert, während die Relativbewegungen zwischen den beiden Verankerungselementen (1, 2) beibehalten werden.
